# EUROPEAN PATENT SPECIFICATION

(11) **EP 1 658 046 B1**
(45) Date of publication and mention of the grant of the patent: **10.12.2008**
(21) Application number: 04740892.7
(22) Date of filing: 09.07.2004
(51) Int. Cl.: A61K 9/00, A61K 38/19, A61K 38/20, A61P 31/20

(54) **MUCOADHESIVE COMPOSITION COMPRISING POLYACRYLATE AND CHEMOATTRACTANT**
MUKOADHÄSIVE ZUSAMMENSETZUNG MIT POLYACRYLAT UND EINEM CHEMOLOCKSTOFF
COMPOSITION MUCOADHESIVE COMPRENANT DU POLYACRYLATE ET UNE SUBSTANCE DE CHIMIO-ATTRACTION

(30) Priority: 31.07.2003 GB 0317999
(43) Date of publication of application: 24.05.2006
(73) Proprietor: Université de Liège, 4031 Angleur (BE)
(72) Inventor: HUBERT, Pascale, B-5300 COUTISSE (BE); EVRARD, Brigitte, B-4537 VERLAINE (BE); DELATTRE, Luc, B-4680 HERMEE (BE); BONIVER, Jacques, B-4802 HEUSY (BE); DELVENNE, Philippe, B-4122 PLAINEVAUX (BE); NOEL, Agnès, B-4653 Bolland (BE); FRANKENNE, Francis, B-4050 Chaudfontaine (BE); FOIDART, Jean-Michel, B-4870 Trooz (BE)
(86) International application number: PCT/EP2004/007617
(87) International publication number: WO 2005/013933

(56) References cited:
- WO-A-97/13502
- WO-A-98/20872
- OH Y-K ET AL: "Enhanced mucosal and systemic immune responses to a vaginal vaccine coadministered with RANTES-expressing plasmid DNA using in situ-gelling mucoadhesive delivery system" VACCINE, BUTTERWORTH SCIENTIFIC. GUILDFORD, GB, vol. 21, no. 17-18, 16 May 2003 (2003-05-16), pages 1989-1997, XP004421114 ISSN: 0264-410X
- DATABASE MEDLINE [Online] US NATIONAL LIBRARY OF MEDICINE (NLM), BETHESDA, MD, US; March 1998 (1998-03), ZHAOPENG H ET AL: "[Improvement of drug bioavailability using protease inhibitors]" XP002297145 Database accession no. NLM9549342 & NIPPON RINSHO. JAPANESE JOURNAL OF CLINICAL MEDICINE. MAR 1998, vol. 56, no. 3, March 1998 (1998-03), pages 595-600, ISSN: 0047-1852

## Description

This invention concerns improvements in or relating to drug delivery systems and has particular reference to genital drug delivery systems.

There is now accumulating evidence that epithelial cells may influence immune reactions in squamous mucosa through the production of cytokines. Granulocyte Macrophage - Colony Stimulating Factor (GM-CSF) produced by keratinocytes acts as a selective chemoattractive molecule for the migration of dendritic cells (DC) into the epithelium and for their differentiation into Langerhans cells (LC) (Kaplan et al, 1992; Pastore et al, 1997). Exposure of LC/DC to GM-CSF *in vitro* prolongs their survival and increases their capacity to present antigens to lymphocytes (Koch et al, 1990). Moreover, in vivo, a correlation has been observed between the amount of GM-CSF produced by some carcinomas and the distribution/differentiation of tumor-associated DC (Colasante et al, 1995). We have previously demonstrated, by using the organotypic raft culture system, that GM-CSF is a potent factor for enhancing the colonization of LC/DC in a (pre)neoplastic epithelium formed in vitro (Hubert et al, 1999). Moreover, the infiltration of organotypic cultures by DC specifically induced the apoptosis of keratinocytes transformed by human papillomavirus (HPV) whereas DC were not affected (Hubert et al, 2001).

These data suggest that the recruitment of DC into a virus-infected and/or (pre)neoplastic epithelium might be beneficial not only by stimulating antiviral and anti-tumor immune responses but also by inducing the death of virus-infected and transformed cells.

Morbidity and mortality caused by HPV infection is a major health problem in developing countries and in the industrialised world, where direct and indirect costs from disease are very high. HPVs are common sexually transmitted pathogens inducing a spectrum of diseases ranging from benign genital warts to invasive carcinoma. Some types of HPV have been shown to be directly involved in the malignant transformation process, especially in the uterine cervix. Up to 99 percent of cervical cancer and its precursors (squamous intraepithelial lesions; SIL) may be attributed to infection by oncogenic human papillomavirus (HPV)(Bosch et al, 1995). However, HPV alone is not sufficient for tumor progression (Herrington, 1995). The role of the intrinsic immunity in controlling HPV infection and the subsequent development of SIL is shown indirectly by the increased frequency of HPV-associated lesions in patients with depressed cell-mediated immunity (Petry et al, 1994; Ellerbrock et al, 2000). Although viral antigens are expressed in a majority of preneoplastic lesions, progression to invasive cancer may occur, suggesting the existence of some qualitative and/or quantitative perturbations in the antigenic presentation function. This hypothesis is reinforced by the observation that most genital warts and SILs are characterized by a decreased density and function of LC compared to the normal paired squamous epithelium (Giannini et al, 2002; McArdle and Muller, 1986; Morelli et al, 1994). Since the immune system clearly plays an important role in influencing the natural history of the disease, there is some evidence that immune response modifiers may have therapeutic value for these lesions. Because of the central role of DC/LC in the induction and regulation of cellular immune responses, pharmacological agents that modulate the recruitment and function of these cells might have clinical interest.

One major problem associated with the genital drug delivery is that the physiological conditions imposed by the protective mechanisms of the cervix or the vagina often lead to a limited contact time of administered drugs with the mucosa and a short duration of therapeutic efficacy, making a frequent dosing regimen necessary. (Robinson and Bologna, 1994; Valenta et al, 2001). The patients are known to tolerate gels better than other conventional dosage forms such as inserts or ointments. Some bioadhesive polymers have attracted considerable attention for their opportunity to prolong the contact of drug with a mucosal surface, without inducing adverse local effects on the epithelium (Richardson at al, 1996).

A use of in situ-gelling mucoadhesive vaginal vaccine delivery systems comprising polycarbophil and poloxamers with a genetic chemokine as adjuvant have been disclosed by Yu-Kyoung in Vaccine 21(2003).

Improvement of drug bioavailability using polycarbophil as protease inhibitors and as absorption enhancer on protein/peptides are disclosed in Medline March 1998 by Zhaopeng H. and al.

The use of polyacrylates mixed with GM-CSF is also teached in WO97/13502A.

According to the invention there is provided a mucoadhesive pharmaceutical composition comprising an acrylic acid containing polymer and a chemoattractant wherein the pH of the composition is 6 or less.

A chemoattractant is generally understood to be a pharmacological agent which modulates the recruitment of immune cells such as a dendritic and/or Langerhans cell. The chemoattractant used in the invention is preferably granulocyte macrophage - colony stimulating factor (GM-CSF, molgramostim), MIP3α (macrophage inflamatory proteine 3α), HβD2 (human β defensin 2), HNP2 (human neutrophil defensin 2) and/or MCP-1 (monocyte chemotactic protein-1). More preferably it is GM-CSF.

A Chemoattractant according to the invention is produced in a bacterial expression system such as E.Coli.

The chemoattractant is present in the pharmaceutical composition at a concentration varying from 300000 to 1000000 International Unit (IU)

The acrylic acid containing polymer is preferably polycarbophil, a synthetic, high molecular weight, polymer of acrylic acid cross-linked with divinyl glycol.

It is more preferably mixed with (97. to 99.9 % of purified water to form a hydrogel The hydrogel has a viscosity between 10000and 110000cP (Brookfield viscosity)

The pH of the composition is preferably 5.5 or less. More preferably it is from 5 to 5.5. The pH of the gel is optimised with alkali such as trometamol, sodium hydroxide or the like.

The composition according to the invention optionally further comprises a preservative and/or stabiliser such as methylparahydroxybenzoate, propylparahydroxybenzoate or the like.

Suitable carriers, excipients and other agents may also be incorporated into the composition to provide improved transfer, delivery and the like.

According to the invention there is also provided a composition according to the invention for use in the treatment of a squamous mucosa, preferably in the treatment of an anogenital or oral disease, especially human papilloma virus.

By anogenital disease, is meant vaginal, cervical, or rectal disease.

According to the invention there is further provided use of a composition according to the invention in the manufacture of a medicament for use in the treatment of a squamous mucosa, preferably in the treatment of an anogenital or oral disease 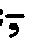 especially human papilloma virus

According to the invention there is also provided a method of treating an anogenital or oral disease which method comprises administering a medically effective amount of a composition according to the invention to a patient in need of such treatment. Typically, treatment with direct administration is done daily, weekly or monthly for a period of time sufficient to reduce, prevent, or ameliorate symptoms

The composition of the invention may also be administered in depot type devices, implants or encapsulated formulation to allow slow or sustained release of the composition.

It has surprisingly been found that an acrylic acid containing polymer, particularly polycarbophil in the form of a hydrogel at pH 5.5 conserves the bioactivity of a chemoattractant molecule (GM-CSF) at 4°C or 37°C for at least 21 days, whereas a decrease in the biological activity of a chemoattractant molecule (GM-CSF) was observed after incorporation in the hydroxypropylcellulose or in the poloxamer hydrogels at both temperatures.

The residual bioactivity of GM-CSF incorporated into both gels at pH 6.9 was surprisingly different. These data could be attributed to ionisation of a part of the carboxyl group present in the polycarbophil gel and absent in the hydroxypropylcellulose and poloxamer formulations.

The GM-CSF bioactivity evaluated after storage in the polycarbophil hydrogel formulated at a pH value of 5.5 was better than that observed for GM-CSF in solution.

These results in the Examples show that the polycarbophil gel (Noveon) is compatible with the diffusion of bioactive GM-CSF with the advantage to stabilize the protein. Moreover, GM-CSF included in this gel formulation is also able to recruit DC into HPV-transformed (pre)neoplastic epithelial tissues *in vitro* and *in vivo.* This formulation which is suitable for genital application will thus restore immune functions which have been shown to be altered in HPV-associated lesions.

The invention is illustrated with reference to the Figures of the drawings in which:

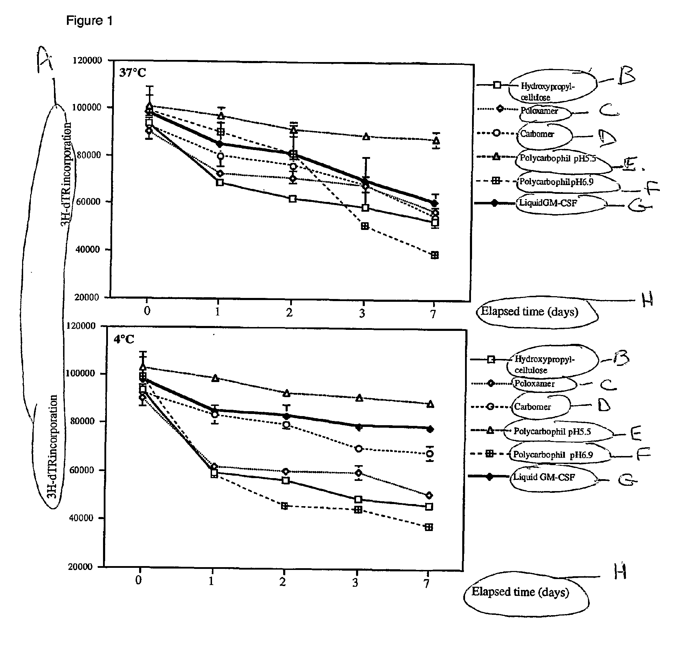

**Figure 1** shows data of residual bioactivity after one week storage and shows graphs of the proliferative response of TF-1 cell line after incubation with GM-CSF extracted at different time intervals (T0 until 1 week) from hydrogels stored at 4°C or at 37°C; growth was measured by 3H-dTR incorporation; each point shows the mean of 6 replicates and bars represent standard errors;

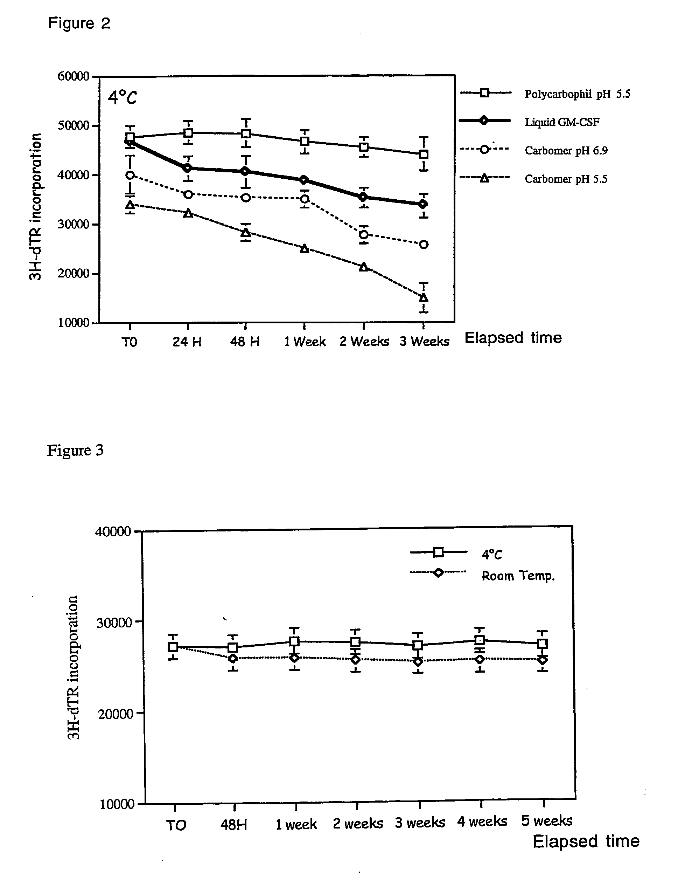

**Figure 2** shows data of residual bioactivity after three weeks storage and shows a graph of the proliferative response of TF-1 cell line after incubation with GM-CSF extracted at different time intervals (T0 until 3 weeks) from hydrogels stored at 4°C; growth was measured by 3H-dTR incorporation; each point shows the mean of 6 replicates and bars represent standard errors;
**Figure 3** shows data of residual bioactivity after five weeks storage of the freeze drying polycarbophil gel and shows a graph of the proliferative response of TF-1 cell line after incubation with GM-CSF extracted at different time intervals from freeze-drying polycarbophil gel pH 5.5 stored at 4°C or at room temperature; growth was measured by 3H-dTR incorporation; each point shows the mean of 6 replicates and bars represent standard errors;

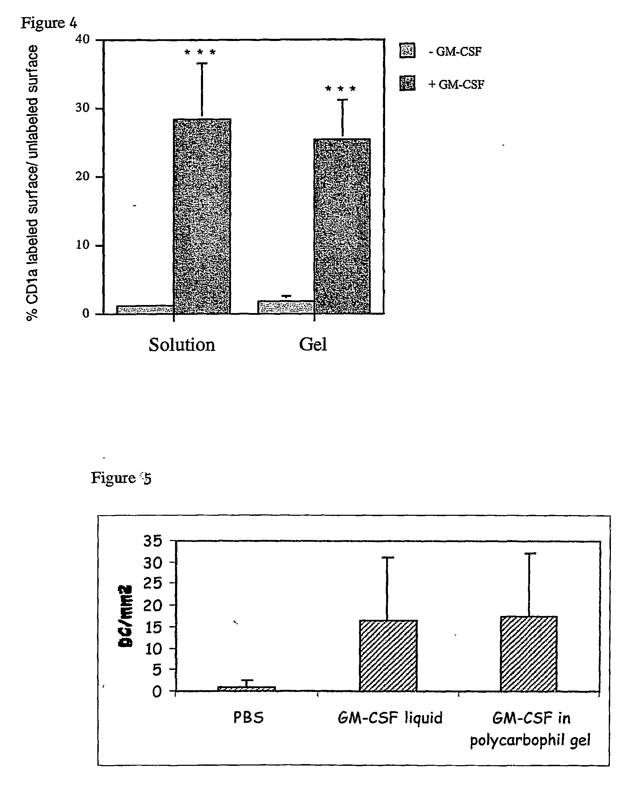

**Figure 4** shows a quantitative evaluation of DC infiltration into organotypic cultures of HPV-transformed keratinocytes under the influence of GM-CSF incorporated or not in polycarbophil gel; the penetration of DC is followed by immunolabeling with anti-CD1a; results are expressed as percentages of surface labeled with anti-CD1a compared to unlabeled surface of the epithelial sheet ± SD (n=5); asterisks indicate statistically significant differences (***: p< 0.001);
Figure 5 shows a quantitative evaluation of DC infiltration into transplants of HPV-transformed keratinocytes; the results are expressed as numbers of DC per mm² of HPV+ epithelium ± SD (n=5 for each condition); asterisks indicate statistically significant differences (***: p< 0.001);

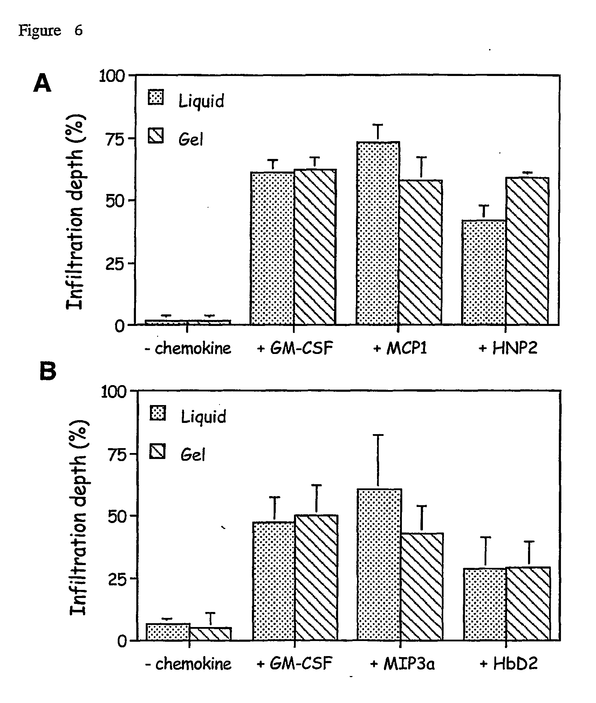

**Figure 6** shows a quantitative evaluation of DC and LC infiltration into organotypic culture of HPV-transformed keratinocytes; the penetration of DC (A) is tested in the absence or in the presence of GM-CSF (used as control), MCP-1 and HNP2 included or not in polycarbophil gel; the infiltration of LC (B) is analysed in the absence or in the presence of MIP3α and HβD2 included or not in the polycarbophil gel; DC and LC were detected by immunolabeling with anti-CD1a antibody; results are expressed as infiltration depth in percentage of epithelial sheet thickness (n=4 for each culture condition).

The present invention is illustrated with reference to the following Examples which are not intended to limit the scope of the claimed invention.

### EXAMPLE 1

In the following Example, Poloxamer P407 (Lutrol) was supplied from BASF (Ludwigshafen, Germany. Carbomer (Carbopol 974P) and Polycarbophil (Noveon AA1) were a gift from Noveon (Brussels, Belgium). Hydroxypropyl cellulose (Klucel GF) was obtained from Hercules (Düsseldorf, Germany). GM-CSF was received from Novartis (Brussels, Belgium). All other chemicals were of analytical grade.

A carbopol gel (1.3% w/w) was prepared by dispersing a carbopol resin in purified water. The mixture was stirred until thickening occurred and then neutralized by dropwise addition of 40% (w/w) tromethamol, until a transparent gel appeared. The quantity of tromethamol was adjusted to achieve a gel pH of 6.9 or 5.5.

A polycarbophil gel (1.0% w/w) was prepared by dispersing the Noveon AA1 in purified water. The mixture was stirred until thickening occurred and then neutralized by dropwise addition of 40% (w/w) tromethamol, until a transparent gel appeared. The quantity of tromethamol was adjusted to achieve a gel pH 6.9 or 5.5.

A poloxamer gel was prepared by using a cold method. Poloxamer P407 20% (w/w) was slowly added under gentle mixing to a pH 6.9 phospate buffer 0.05M at a temperature of 4°C. The mixture was allowed to dissolve overnight at 4°C until a clear solution was obtained. Finally, hydroxypropylcellulose 7 %(w/w) was slowly added to a pH 6.9 phosphate buffer 0.05M under gentle mixing until a transparent gel appeared.

### EXAMPLE 2

A GM-CSF bioassay was carried out using TF-1 cells.

900 pg of GM-CSF, diluted in RPMI (culture medium)with 2% FCS (foetal calf serum), were incorporated per gram of different gels by gently mixing. The gels were kept for different periods of time at obscurity and at 37°C or 4°C. The gels were then diluted with 6 ml of RPMI 2% FCS in order to extract the GM-CSF. The final concentration of GM-CSF was 150 ng/ml. This concentration was chosen in order to be in the exponential growth curve of TF1 cells (data not shown). After a filtration on 0.45 µm pore filters (Millex-HV, Millipore, Bedford, MA), solutions were stored at - 20°C before the bioassay. GM-CSF in solution (150 ng/ml) was also kept during the different intervals of time at 37°C and 4°C and filtered before storage.

For stability experiments after freeze-drying, one gram of polycarbophil gel pH 5.5 containing 900 pg of GM-CSF was filled in 10 ml glass vials. The samples were placed into the drying chamber of an HETO FD8 freeze-dryer (Jouan Nordic A/S, Allerød, Denmark) and pre-cooled at -35°C during 3h30. Drying was performed at a pressure of 0.8 bar for 3h at -15°C and at a pressure of 0.01 bar for 12h. After this period, a secondary drying step for 5h at 25°C was applied before the vials were sealed and removed. The lyophilized gels were kept for different periods of time at obscurity and at room temperature or 4°C. The freeze-dried samples were then resuspended in one ml of demineralised water before being diluted with 6 ml of RPMI 2% FCS in order to extract the GM-CSF.

The factor-dependent human cell line TF-1 (ATCC, CRL-2003) was cultivated following previously published recommendations (20) with minor modifications. Briefly, the cells were incubated in RPMI 1640 medium containing 10% fetal bovine serum and 5 ng/ml GM-CSF at 37°C 5% CO2. Cells were deprived of GM-CSF for 24 hours and were cultured in the presence of 2% FCS. The cells were plated in a round-bottom 96 well microtiterplate at 50,000 cells per well, in 100 µl RPMI 2% FCS and 100 µl of GM-CSF included in the different polymers for various intervals of time. The tests were performed in quintuplate. The cultures were incubated for 24 hours in the presence of 3H-TdR (2 µCi/well, 7 Ci/mmol, Moravek Biochemicals, CA). Cells were harvested by an automated sample harvester (Packard, Canberra, Tilburg, The Netherlands) and counted in a liquid scintillation counter (Top Count, Packard, Canberra).

### EXAMPLE 3

In the following Example the preparation of a Dendritic cell culture and labeling with lipophilic fluorescent cell tracer is described.

DC were generated from the adherent fraction of human PBMC (Peripheral Blood Mononuclear Cells) with 800 U/ml GM-CSF (Novartis, Brussels) and 20 U/ml IL-4 (Biosource Europe, Nivelles, Belgium), as previously described (16). DC generated for this study constituted a 90% pure cell population based on several criteria including morphology, forward-scatter and side-scatter values observed by FACS (Fluorescence Activated Cell Sorter) analysis and surface phenotype (CD1a+, CD 14-, CD4+, CD54+, ...).

The DC were labeled with a lipophilic fluorescent marker (CM-DiL, Molecular Probes, Leiden, The Netherlands) according to a previously described procedure with minor modifications. Briefly, 4 x 106 DC were resuspended in 1 ml of PBS and heated to 37°C. CM-DiL was diluted in 1 ml of PBS preheated to 37°C to obtain a final concentration of 16 µg/ml. The dye was mixed for several seconds until the dye was evenly distributed. This solution was immediately transferred to the cell suspension and rapidly mixed by pipetting. The cells were incubated for 2 min at 37°C, then for 2 min on ice. Finally, they were transferred in 40 ml of PBS at 4°C, centrifuged and resuspended in the appropriate medium.

### EXAMPLE 4

In the following Example, the preparation of a Langerhans cell culture is described.

Cord blood mononuclear cells (CB MNC) were recovered after discontinuous density gradient centrifugation using LymphoprepTM (Nycomed Pharma As, Oslo, Norway) within 24 hours after collection. CD34+ cells were isolated from CB MNC using the MACS Direct CD34 Progenitor Cell Isolation Kit (Miltenyi Biotec GmBH, Bergisch Gladbach, Germany) and MiniMACS separation columns (Miltenyi Biotec) according to the manufacturer's protocol. Fifteen 104 CD34+ cells were seeded in T25 flasks (Sarstedt, Inc, Newton, NC) in 10 ml of RPMI 1640 medium supplemented with 10% FCS, antibiotics and 50 □M mercaptoethanol (all from GIBCO-BRL, Gaithersburg, MO). Cultures were supplemented with previously optimized concentrations of the following human molecules: SCF (20 ng/ml, specific activity (SA) > 5 105 U/mg), TPO (10 U/ml, SA >1 106 U/mg), FIT3-L (25 ng/ml, SA >2 105 U/mg), GM-CSF (200 U/ml, SA: 11,1 106 U/mg), TNF□ (50 U/ml, SA >2 107 U/mg), IL4 (100 U/ml, SA >2 106 U/ml) and TGF-□1 (5 ng/ml or 12.5 ng/ml, SA >2 108 U/mg). All these agents were purchased from PeproTech (Rockey Hill, NJ), except for GM-CSF and IL4 which were obtained from Amoytop (Amoytop Biotech, Xiamen, China) and Biosource (Nivelles, Belgium), respectively. The cells were cultured at 37°C in a humidified atmosphere and in the presence of 5% CO2. At day 7, the cellular density was adjusted to 2x10⁴ per cm2 and the cells were fed, at days 7 and 14, with GM-CSF, IL4, TNFα and TGF-β1 at the same concentrations as those used at the start of the culture, except for TGF-β1 at day 14 which was 12.5 ng/ml for all the cultures. At day 18, cells were collected from cultures by vigorous pipetting to prepare single cell populations.

### EXAMPLE 5

In the following Example, the preparation of an organotypic culture is described.

Organotypic cultures of HPV-transformed keratinocytes (SiHa cell line (10)) were prepared as previously reported (17). After a 2 week stratification of keratinocytes, labeled DC were seeded on top of the in vitro formed epithelium at a concentration of 2x10⁵ cells/50 µl of culture medium. The organotypic culture was then placed on the polycarbophil gel containing or not 800U/ml of GM-CSF. In order to maintain the culture, the polycarbophil gel (1,5 w/w) was prepared by dispersing the Noveon AA1 in keratinocyte growth medium. The mixture was neutralized by dropwise addition of 40% (w/w) tromethamol. The positive control was a solution of liquid GM-CSF at 800U/ml. In additional experiments, 500 ng/ml of Monocyte Chemotactic Protein 1 (MCP-1, PeproTech, Rocky Hill, NJ), 750 ng/ml of Human Neutrophil Peptide-2 (HNP2, Sigma-Aldrich, St Louis, MO), 750 ng/ml of Human β Defensin -2 (HβD-2, PeproTech, Rocky Hill, NJ) or 500 ng/ml of Macrophage Inflammatory Protein 3α (MIP3α, PeproTech, Rocky Hill, NJ) were also tested as chemoattractant included in liquid culture medium or in the polycarbophil gel at the same concentration. After 48 h at 37°C, the collagen rafts were harvested. The cultures were then embedded in O.C.T. compound (Tissue Tek, Sakura, Netherlands) at -70°C and sectioned with a cryostat. The organotypic cultures were counterstained with the fluorescent dye 4',6-Diamidine-2-phenylindole Dihydrochloride (DAPI, Roche Diagnostics, Brussels, Belgium). Infiltration of fluorescent DC was visualized with a fluorescent microscope (Leica DMLB microscope, Heidelberg GmBH, Germany) equipped with a 40x objective.

### EXAMPLE 6

In the following Example the methodology used for assessment of CD1a+ cell infiltration in organotypic cultures is described.

The density of DC migrating into the epithelial layer was assessed by the avidin-biotin-peroxidase technique (Vectastain ABC kit, Vector laboratories, Burlingame, CA) with an anti-CD1a monoclonal antibody (clone NA1/34 from Dako, Glostrup, Denmark). Nine µm frozen sections were fixed in cold acetone for 3 minutes and endogenous peroxidases were blocked with 0.1% H2O2 for 30 minutes. Sections were then incubated sequentially with anti-CD1a antibody (at a 1/40 dilution in PBS 2% BSA, 0.01 M NaN3) or with an isotype-matched control antibody for 1 h, with a biotinylated mouse anti-Ig antibody for 30 min and with streptavidin HRP AB Complex for another 30 minutes. Positive cells were visualized by a 3,3'-diaminobenzidin substrate (DAB). The sections were counterstained with hematoxylin. The DC infiltration in organotypic cultures was evaluated by measuring the surface of immunostained cells with a computerized system of image analysis (CAS, Becton Dickinson, Erembodegem, Belgium) following a method previously described (6). The entire surface of each culture section was analyzed and 5 sections of each culture were stained with anti-CD1a. The positive surface was expressed as the percentage of the total surface analyzed. In additional experiments, the LC/DC infiltration depth in the organotypic culturs was evaluated by measuring the distance between the top of the epithelium and individual DC with a computerized system of image analysis (CAS, Becton Dikinson, Erembodegem, Belgium). We then calculated the ratio between the infiltration depth and the thickness of the culture. The infiltration depth of all LC/DC observed in a culture section was analyzed and 4 sections of each culture were stained with anti-CD1a antibody. The percentage ratio was 0% for LC/DC staying on the top of the culture and 100% for LC/DC reaching the bottom of the epithelium. Statistical analysis was performed by using the Student t test (Instat Mac 2.01 software; Graph-Pad Software, San Diego, CA).

### EXAMPLE 7

In the following Example the methodology used for the Xenograft assay in NOD/SCID mice is described.

HPV-transformed keratinocytes (2x10⁵, CasKi cell line, ATCC, CRL-1550) were plated on collagen gel (2mg/ml of type I collagen isolated from rat tail tendons) inserted in Teflon rings (Renner, Dannstadt, Germany) and maintained in culture for one day before transplantation onto mice. Twelve- week-old NOD-scid/scid mice were used for this study. They were housed under sterile conditions and water and feed were provided ad libitum. Before transplantation, the cells were washed and the medium was drained. The cell-coated collagen gels were then covered with a silicone transplantation chamber (Renner, Dannstadt, Germany) and implanted *in toto* onto the dorsal muscle fascia of mice as previously described (2, 11). The mice were killed by cervical dislocation 24 days later. The transplants were excised, embedded in O.C.T. compound (Tissue Tek, Sakura, Netherlands) and frozen on dry ice for cryostat sectioning. In DC recruitment experiments, 2x10⁶ fluorescent-labeled DC were intravenously injected at day 21 and PBS, polycarbophil gel alone or with chemoattractants (GM-CSF liquid: 4x10⁵ U/ml; GM-CSF incorporated in polycarbophil gel: 4x10⁵ U/g of gel) were injected twice (200 µl each) into the transplantation chamber (24 and 48 hours before the mice were sacrificed). Transplant sections were counterstained with the fluorescent dye 4',6-Diamidine-2-phenylindole Dihydrochloride (DAPI, Roche Diagnostics, Brussels, Belgium). Infiltration of fluorescent DC was visualized with a fluorescent microscope (Leica DMLB microscope, Heidelberg GmBH, Germany) equipped with a 40x objective. Fluorescent DC were counted in the epithelium and, after anti-keratin immunolabeling, the epithelial surface was evaluated with a computerized system of image analysis (CAS, Becton Dickinson, Erembodegem, Belgium). Infiltration results were expressed as numbers of DC per mm² of epithelium. Statistical analysis was performed by using the Student t test (Instat Mac 2.01 software; Graph-Pad Software, San Diego, CA).

### EXAMPLE 8

In the following Example, the methodology used in immunofluorescent labelling is described.

Graft cryosections (5 µm in thickness) were fixed in acetone at -20°C and in 80% methanol at 4°C. They were then incubated for 60 min at room temperature with the primary antibodies: anti-mouse type IV collagen (SIF105 rabbit anti-mouse, gift of Dr A. Noel) and anti-human keratin (KL1, mouse anti-human, Immunotech, Marseille, France). The appropriate secondary antibodies were then applied for 30 min: swine anti-rabbit conjugated to Texas red (Dako, Glostrup, Denmark) and sheep anti-mouse conjugated to fluorescein-isothiocyanate (Sigma-Aldrich). For double immunofluorescence-labeling studies, sections were first incubated with the two primary antibodies and then with FITC- and Texas red-conjugated secondary antibodies. After washes in PBS, coverslips were mounted and labeling was analysed under an microscope equipped with epifluorescence optics.

### EXAMPLE 9

In the following Example, the measurement of GM-CSF bioactivity following incorporation into different polymers at 4°C and 37°C using a bioassay with TF-1 cells is described and analysed.

The GM-CSF biological activity after incorporation into the different hydrogels was measured at different intervals of time using a bioassay of growth stimulation with the GM-CSF-dependant TF-1 cell line. The injectable solution (Leucomax, Novartis, Brussels, Belgium) was chosen as positive control. The four polymers were tested at a pH value of 6.9 which was reported to be optimal for GM-CSF stability and which correspond to the pH value of the injectable solution (data from Novartis). As polycarbophil was found to be a particularly convenient polymer for cervico-vaginal formulations and as it is usually recommended to develop cervico-vaginal formulations at acidic pH values, the polycarbophil hydrogel was also tested at pH 5.5.

As shown in Figure 1, there was a decrease in the bioactivity of the injectable solution of GM-CSF more marked at 37°C than at 4°C which is in agreement with previous data (30). A marked reduction of GM-CSF activity after incorporation in the hydroxypropylcellulose (Klucel) or in the poloxamer hydrogel (Lutrol) at both temperatures was also observed. In contrast, polycarbophil gel (Noveon) pH 5.5 induced a protection against GM-CSF degradation. However, this protective effect was not observed at pH 6.9. Carbomer (Carbopol) conserved an activity of GM-CSF similar to that observed with the injectable solution at both temperatures and during at least a week.

Additional experiments were performed in order to evaluate the protective effect of the polycarbophil gel during a period of more than one week. At the same time, the impact of a pH reduction on the GM-CSF stability in the carbomer gel was tested. As shown in Figure 2, the bioactivity of the injectable solution of GM-CSF, used as reference, decreased regularly during the 3 weeks, which is in agreement with literature data (30). As previously observed, a protective effect of polycarbophil gel pH 5.5 was found during the first week of storage. Interestingly, the GM-CSF bioactivity remained stable over 3 weeks and was more marked in the polycarbophil gel pH 5.5 than with the injectable solution. Carbomer conferred an activity of GM-CSF similar to that observed with the injectable solution during at least three weeks, but surprisingly, the pH reduction to 5.5 induced a rapid loss in activity when GM-CSF was incorporated in Carbomer. Similar observations were made when the hydrogels were stored at 37°C (data not shown).

These results suggest that the polycarbophil (Noveon) at pH 5.5 could be convenient to topically apply GM-CSF on a squamous genital mucosa.

### EXAMPLE 10

Freeze-drying is a well-established storage method under sterile conditions. The bioactivity of GM-CSF after lyophylisation of polycarbophil gel and after storage at different time intervals and different temperatures was tested. After lyophylisation, the residues appeared voluminous and snow-like. The lyophylisates were re-suspendable in water. However, it was necessary to take additional actions such as shaking and vortexing to achieve full re-suspension after addition of one ml of water for one gram of gel. After successful re-suspension, the bioactivity of GM-CSF was reexamined and compared to that obtained after non-lyophylisated sample (Figure 3). The freeze-drying was found to prevent loss of activity for a period of at least 5 weeks, whatever the temperature of storage.

These results further confirm that the polycarbophil (Noveon) at pH 5.5 could be convenient to topically apply GM-CSF on a squamous genital mucosa.

### EXAMPLE 11

In the following Example, the influence of GM-CSF incorporated in polycarbophil gel on the recruitment of DC in *in vitro* formed (pre)neoplastic epithelium is demonstrated.

It was investigated whether GM-CSF included in a polymer modulates the ability of DC to infiltrate an *in vitro*-formed (pre)neoplastic epithelium, reminiscent of high-grade (pre)neoplastic lesions observed *in vivo.* Monocytic precursors from the plastic adherent fraction of human peripheral blood mononuclear cells cultivated in the presence of 800 U/ml of GM-CSF and 20 U/ml of IL-4 for 7 days showed a morphology of DC with abundant dendritic membrane protrusions (data not shown). By FACS analysis , these cells also displayed typical phenotypical features of DC with the presence of CD1a, CD4, CD86 and CD54 (ICAM-1) antigens, a moderate expression of class II MHC (HLA-DR) molecules and the absence of macrophage (CD14), B (CD20) and T (CD3) cell markers. DC were layered on top of organotypic cultures of HPV-transformed keratinocytes in the presence of GM-CSF incorporated or not in the polycarbophil gel. The effect of GM-CSF on DC infiltration was assessed by examining cryosections of organotypic cultures frozen 48 hours after GM-CSF addition. The ability of the GM-CSF gel to modulate the infiltration of DC was determined by the evaluation of fluorescent DC into the organotypic cultures. Figure 4 illustrates representative experiments showing the density of fluorescent-labeled DC in organotypic cultures of HPV-transformed keratinocyte incubated with or without exogenous GM-CSF incorporated or not in polycarbophil gel. DC layered onto organotypic cultures of SiHa poorly infiltrated the epithelial cell layers in the absence of GM-CSF , whereas GM-CSF in solution or incorporated in the gel caused a significant increase in the density of DC observed within the epithelial sheet. Quantitative analysis of DC infiltration was performed on organotypic cultures derived from the HPV-transformed cell line SiHa . The density of DC in the *in vitro*-formed epithelium was quantified by evaluating the surface labeled with the anti-CD1a antibody, throughout the full thickness of organotypic cultures. Under basal conditions (in the absence of GM-CSF, liquid or incorporated in the gel), the level of DC infiltration in the cultures was low (1.64 ± 0.78 % of CD1a labeled surface). When the medium of organotypic cultures of HPV+ cell lines was supplemented with GM-CSF, the infiltration of DC significantly improved (28.33 ± 8.11 % of CD1a labeled surface) and the density of DC observed in these conditions was similar to that induced by the GM-CSF included in the gel (25.38± 5.81 % of CD1a labeled surface).

### EXAMPLE 12

In the following Example the influence of the inclusion of GM-CSF in polycarbophil gel on the stimulation of the migration of human DC in xenografts of HPV-transformed keratinocytes was investigated.

In order to address the *in vivo* efficacy of GM-CSF included in polycarbophil gel, HPV-transformed keratinocytes transplanted cultured on a collagen gel were onto the dorsum of NOD-SCID mice. Twenty-four days after transplantation, mice were killed and the grafts were removed for immunohistological analysis. Histological examination of thin sections revealed that the growth pattern of the grafts reproduces a high-grade squamous (pre)neoplastic lesion and the collagen gel was replaced by a granulation tissue. Transplantation of HPV+ keratinocytes induced an angiogenic response in the host tissue starting from vessels of the dorsal muscle and subsequently extending far up into the collagen gel. The vessels sprouted into the tumor epithelium, which started to invade the newly formed granulation tissue 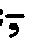

To demonstrate the efficacy of GM-CSF incorporated in polycarbophil gel, two groups of five mice were treated by injecting 200 µl of GM-CSF, in PBS or in the gel, in the transplantation chamber covering the (pre)neoplastic epithelium, every day, for a period of two days prior the killing of the mice. A control group of mice was treated with PBS alone. At the first application of GM-CSF, 2 x 106 human DC pre-labeled with fluorescent tracer were intravenously injected in the mice. The grafts were harvested one day after the last administration of GM-CSF and processed for microscopic examination. The DC infiltration in transplants was assessed by looking for stained DC in histological sections. As shown in Figure 5, the applications of GM-CSF in the transplantation chamber resulted in a significant (p<0.001) recruitment of DC in the tumor grafts of treated animals, while DC were rarely detected in the epithelium of control mice. Quantitative analysis of DC infiltration was performed by determining the number of DC per mm2 of epithelial tissue. The DC infiltration in the grafts was very low (1.1 ± 1.4 DC/mm2) in the absence of GM-CSF and drastically increased after treatment with liquid GM-CSF (16.6 ± 14.6 DC/mm2). Similar results were observed when GM-CSF was incorporated into polycarbophil gel (17.5 ± 14.6 DC/mm2).

### EXAMPLE 13

In the following Example the ability of other chemoattractant molecules, incorporated in the polycarbophil gel, to induce the infiltration of DC and Langerhans cells (LC) in *in vitro* formed (pre)neoplastic epithelium was investigated.

Since GM-CSF may not be the only factor implicated in the control of LC/DC intra-epithelial migration (12), we wanted to identify other molecules able to stimulate the migration of LC/DC, and/or whose the production could be affected in HPV+ keratinocytes.

Both MIP3α and HβD2 (human β defensin 2) have been shown to be chemotactic for immature dendritic cells through the interaction with CCR6 (44, 41, 39, 42). Interestingly, SILs have been associated with an intermittent pattern of staining for MIP3α compared with normal exocervix. HNP2 (human neutrophil defensin 2) selectively induces the migration of immature human DC independent of the CCR6 receptor (43). MCP-1 plays an important role in the recruitment of LC/DC and monocytes (38) and an inverse correlation between the expression of the MCP-1 gene and the HPV oncogenes E6/E7 was described in cervical carcinoma cell lines (40). Since monocytes-derived DC are deficient for the CCR6 (37) the receptor for MIP3α and HβD-2, we have developed a protocol which allowed to generate a large number of LC from CD34+ precursors (Hubert et al, submitted).

We investigated whether the addition of MIP3α and HβD-2, or MCP-1 and HNP2 could modulate the ability of LC and DC respectively to infiltrate an in vitro-formed (pre)neoplastic epithelium, reminiscent of a cervical high-grade SIL observed *in vivo.*
DC or LC were layered on the top of these cultures with or without chemoattractant molecules included or not in polycarbophil gel. LC/DC infiltration was assessed by examining immunohistological sections of organotypic cultures at 48 hours following chemoattractant addition.

The migration of LC/DC in the in vitro-formed epithelium was quantified by measuring the infiltration depth of all cells labeled with the anti-CD1a antibody throughout the full thickness of organotypic cultures. LC/DC poorly infiltrated the epithelial layer in the absence of GM-CSF (Figure6), whereas addition of GM-CSF caused a significant increase in the density of DC observed in the epithelial layer. LC/DC migrated in a similar manner in the presence of GM-CSF included or not in the polycarbophil gel.

When the medium of organotypic culture was supplemented with MCP1 or HNP2 (Figure 6A), the infiltration of DC improved compared to the basal infiltration without chemoattractant, and reached an infiltration level equivalent to that obtained with GM-CSF. The inclusion of the chemoattractant molecules in the polycarbophil gel did not modify the DC infiltration compared to that observed in the presence of the solution of chemoattractant.

Similar results were obtained with LC and when the medium of organotypic culture was supplemented with MIP3□ or H□D-2 (Figure 6B).

These results show that MIP3α, HβD-2, MCP-1 and HNP2 included in a polycarbophil gel could also be used to recruit LC/DC in mucosal surfaces and be useful as a new immunotherapeutic approach for genital HPV-associated (pre)neoplastic lesions.

### EXAMPLE 14

In the following example,a formulation of pharmaceutical composition comprising GM-CSF,as an active substance has been prepared by mixing the active substance in purified water with 1.4% polycarbophil, 0.08% methylhydroxybenzoate and 0.02% propylparahydroxybenzoate. The pH of the formulation is then regulated with Trometamol at pH5.5

### EXAMPLE 15

In the following example,a formulation of pharmaceutical composition comprising Molgramostin, as an active substance has been prepared by mixing the active substance in purified water with 1.4% polycarbophil, 0.08% methylhydroxybenzoate and 0.02% propylparahydroxybenzoate. The pH of the formulation is then regulated with Trometamol at pH5.5

### REFERENCES

1. Bai, J., L. Chang, and J. Guo. 1996. Effects of polyacrylic polymers on the degradation of insulin and peptide drugs by chymotripsin and trypsin. J. Pharm. Pharmacol. 48:17-22.
2. Bajou, K., A. Noël, R.D. Gerard, V. Masson, N. Brunner, C. Holst-Hansen, M. Skobe, N.E. Fusenig, P. Carmeliet, D. Collen, and J.M. Foidart. 1998. Absence of host plaminogen activator inhibitor 1 prevents cancer invasion and vascularization. Nature Medicine. 4:923-928.
3. Bosch, F.X., M.M. Manos, N. Munoz, M. Sherman, A.M. Jansen, and J. Peto. 1995. Prevalence of human papillomavirus in cervical cancer: a worldwide perspective. International biological study on cervical cancer (IBSCC) study Group. J. Natl. Cancer Inst. 87:796-802.
4. Chang, J., Y. Oh, H. Choi, and Y. Kim. 2002. Rheological evaluation of thermosensitive and mucoadhesive cervico-vaginal gels in physiological conditions. Int. J. Pharm. 241:155-163.
5. Colasante, A., G. Castrilli, F. Bianca, M. Brunetti, and P. Musiani. 1995. Role of cytokines in distribution and differentiation of dendritic cell/langerhans'cell lineage in human primary carcinomas of the lung. Hum. Pathol. 26:866-872.
6. Delvenne, P., W. Al-Saleh, C. Gilles, A. Thiry, and J. Boniver. 1995. Inhibition of growth of normal and human papillomavirus-transformed keratinocytes in monolayer and organotypic cultures by interferongamma and tumor necrosis factor-alpha. Am. J. Pathol. 146:589-598.
7. Delvenne, P., P. Hubert, N. Jacobs, S.L. Giannini, L. Havard, I. Renard, D. Saboulard, and J. Boniver. 2001. The organotypic culture of HPV-transformed keratinocytes : an effective in vitro model for the development of new immunotherapeutic approaches for mucosal (pre) neoplastic lesions. Vaccine 19:2557-2564.
8. Ellerbrock, T.V., M.A. Chiasson, T.J. Bush, X.W. Sun, D. Sawo, K. Brudney, and T.C. Wright. 2000. Incidence of cervical squamous intraepithelial lesions in HIV-infected women. JAMA 283:1031-1037.
9. Eouani, C., P. Piccerelle, P. Prinderre, E. Bourret, and J. Joachim. 2001. In-vitro comparative study of buccal mucoadhesive performance of different polymeric films. Eur. J. Pharm. Biopharm. 52:45-55.
10. Friedl, F., I. Kimura, T. Osato, and Y. Ito. 1970. Studies on a new human cell line (SiHa) derived from carcinoma of uterus. I. Its establishment and morphology. Proc. Soc. Exp. Biol. 135:543-545.
11. Fusenig, N.E. 1994. In I. Leigh, B., Lane and F. Watt (ed.) The Keratinocyte Handbook. Cambridge Univ. Press, Cambridge.
12. Gauger, L.J. 1983. Extemporaneous preparation of a dinoprostone gel for cervical ripening. Am. J. Hospi. Pharm. 40:2195-2196.
13. Giannini, S.L., P. Hubert, J. Doyen, J. Boniver, and P. Delvenne. 2002. Influence of the mucosal epithelium microenvironment on Langerhans cells: implications for the development of squamous intraepithelial lesions of the cervix. Int. J. Cancer 10:654-659.
14. Herrington, C.S. 1995. Human papillomaviruses and cervical neoplasia. II. Interaction of HPV with other factors. J. Clin. Pathol. 48:1-6.
15. Hubert, P., S.L. Giannini, A. Vanderplasschen, R. Greimers, E. Franzen-Detrooz, N. Jacobs, J. Boniver, and P. Delvenne. 2001. Dendritic cells Induce the Death of Papillomavirus-transformed Keratinocytes. FASEB J. 15:2521-2523.
16. Hubert, P., R. Greimers, E. Franzen-Detrooz, J. Doyen, P. Delanaye, J. Boniver, and P. Delvenne. 1998. In vitro propagated dendritic cells frompatients with human-papilloma virus-associated preneoplastic lesions of the uterine cervix: use of Flt3 ligand. Cancer Immunol. Immunother. 47:81-89.
17. Hubert, P., F. van den Brule, S.L. Giannini, E. Franzen-Detrooz, J. Boniver, and P. Delvenne. 1999. Colonization of in vitro-formed cervical human papillomavirus- associated (pre)neoplastic lesions with dendritic cells: role of granulocyte/macrophage colony-stimulating factor. Am. J. Pathol. 154:775-784.
18. Jacobs, N., M.P. Moutschen, E. Franzen-Detrooz, V. Boniver, J. Boniver, and P. Delvenne. 1998. Organotypic culture of HPV-transformed keratinocytes: a model for testing lymphocyte infiltration of (pre) neoplastic lesions of the uterine cervix.. Virchows Arch.432:323-330.
19. Kaplan, G., G. Walsh, L.S. Guido, P. Meyn, R.A. Burkhardt, R.M. Abalos, J. Barker, P.A. Frindt, T.T. Fajardo, R. Celona, and Z.A. Cohn. 1992. Novel responses of human skin to intradermal recombinant granulocyte/macrophage-colony-stimulating factor: Langerhans cell recruitment, keratinocyte growth and enhanced wound healing. J. Exp. Med. 175:1717-1728.
20. Kitamura, T., T. Tange, T. Terasawa, S. Chiba, T. Kuwaki, K. Miyagawa, Y.F. Piao, K. Miyazono, A. Urabe, and F. Takaku. 1989. Establishment and characterization of a unique human cell line that proliferates dependently on GM-CSF, IL-3, or erythropoietin. J. Cell. Physiol. 140:323-334.
21. Knuth, K., M. Amiji, and J. Robinson. 1993. Hydrogel delivery system for cervico-vaginal and oral applications: formulations and biological consideration Adv. Drug Deliv. Rev. 11:137-167.
22. Koch, F., C. Heufler, E. Kampgen, D. Schneeweiss, G. Bock, and G. Schuler. 1990. Tumor necrosis factor α maintains the viability of murine epidermal Langerhans cells in culture, but in contrast to granulocyte/macrophage colony-stimulating factor, without inducing their functional maturation. J. Exp. Med. 171:159-165.
23. Lynas, K.W. 1980. Preparation of prostaglandin F2alpha cervico-vaginal gel. Austral. J. Hospi. Pharma. 10:88-92.
24. MacKenzie, I.Z., A.J. Davies, and M.P. Embrey. 1979. Fetal death in utero managed with cervico-vaginal prostaglandin E2 gel. B.M.J. 1:1764-1765.
25. McArdle, J.P., and H.K. Muller. 1986. Quantitative assessements of Langerhans' cells in human cervical intraepithelial neoplasia and wart virus infection. Am. J. Obstet. Gynecol. 154:509-515.
26. Morelli, A.E., G. Belardi, G. DiPaola, A. Paredes, and L. Fainboim. 1994. Cellular subsets and epithelial ICAM-1 and HLA-DR expression in human papillomavirus infection of the vulva. Acta. Derm. Venereol. 74:45-50.
27. Pastore, S., E. Fanales-Belasio, C. Albanesi, L.M. Chinni, A. Giannetti, and G. Girolomoni. 1997. Granulocyte macrophage colony-stimulating factor is overproduced by keratinocytes in atopic dermatitis. J. Clin. Invest. 99:3009-3017.
28. Peppas, N.A., and J.J. Sahlin. 1996. Hydrogels as mucoadhesive and bioadhesive materials. Biomaterials 17:1553-1561.
29. Petry, K.U., D. Scheffel, U. Bode, T. Gabrysiak, H. Koche, E. Kupsch, M. Glaubitz, S. Niesert, H. Kuhnle, and I. Schedel. 1994. Cellular immunodeficiency enhances the progression of human papillomavirus-associated cervical lesions. Int. J. Cancer 57:836-840.
30. Pettit, D.K., J.R. Lawter, W.J. Huang, S.C. Pankey, N.S. Nightlinger, D.H. Lynch, J.A. Schuh, P.J. Morrissey, and W.R. Gombotz. 1997. Characterization of poly(glycolide-co-D,L-lactide)/poly(D,L-lactide) microspheres for controlled release of GM-CSF. Pharm. Res. 14:1422-1430.
31. Renard, I., D. Mezzanzanica, S. Canevari, S. Ferrini, J. Boniver, P. Delvenne, and N. Jacobs. 2002. Anti-CD3/anti epidermal growth factor receptor-bisoecific antibody retargeting of lymphocytes against human neoplastic keratinocytes in an autologous organotypic culture model. Am. J. Pathol. 160:113-122.
32. Richardson, J., J. Whetstone, A. Fisher, P. Watts, N. Farraj, M. Hinchcliffe, L. Benedetti, and L. Illum. 1996. Gamma-scintigraphy as a novel method to study ditribution and retention of a bioadhesive cervico-vaginal delivery in sheep. J. Control. Release 42:133-142.
33. Robinson, R., and W. Bologna. 1994. Cervico-vaginal and reproductive system treatments using a bioadhesive polymer. J. Control. Release 28:88-94.
34. Shawesh, A., S. Kallioinene, L. Hellen, O. Antikainen, and J. Yliruusi. 2002. Pluronic F-127 gels as a vehicle for topical formulations of indomethacin and rheological behaviour of these formulations. Pharmazie 57:186-190.
35. Valenta, C., C. Kast, I. Harich, and C. Bernkop-Schnurch. 2001. Development and in vitro evaluation of a mucoadhesive cervico-vaginal delivery system for progesterone. J. Control. Release 77:323-332.
36. Viac, J., Y. Chardonnet, M.C. Chignol, and D. Schmitt. 1993. Papilloma viruses, warts, carcinoma and Langerhans cells. In vivo 7:207-212.
37. Dieu, M., B. Vanbervliet, A. Vicari, J. Bridon, E. Oldham, S. Ait-Yahia, F. Briere, A. Zlotnik, S. Lebecque, and C. Caux. 1998. Selective recruitment of immature and mature dendritic cells by distinct chemokines expressed in different anatomic sites. J. Exp. Med. 188: 373-386.
38. Nakamura, K., I. Williams, and T. Kupper. 1995. Keratinocyte-derived monocyte chemoattractant protein 1 (MCP-1): analysis in a transgenic model demonstrates MCP-1 can recruit dendritic and Langerhans cells to skin. J. Invest. Dermatol. 105: 635-643.
39. Power, C., D. Church, A. Meyer, S. Alouani, A. Proudfoot, I. Clark-Lewis, S. Sozzani, A. Mantovani, and T. Wells. 1997. Cloning and characterization of a specific receptor for the novel CC chemokine MIP-3alpha from lung dendritic cells. J. Exp Med. 186: 825-835.
40. Riethdorf, S., L. Riethdorf, N. Richter, and T. Ning. 1998. Expression of the MCP-1 gene and the HPV 16 E6/E7 oncogenes in squamous cell carcinomas of the cervix uteri and metastases. Pathobiology 66: 260-267.
41. Sozzani, S., F. Sallusto, W. Luini, D. Zhou, L. Piemonti, P. Allavena, J. Van Damme, S. Valitutti, A. Lanzavecchia, and A. Mantovani. 1995. Migration of dendritic cells in response to formyl peptides, C5a, and a distinct set of chemokines.. J. Immunol. 155: 3292-3295.
42. Sozzani, S., W. Luini, A. Borsatti, N. Polentarutti, D. Zhou, L. Piemonti, G. D'Amico, C. Power, T. Wells, M. Gobbi, P. Allavena, P., and A. Mantovani. 1997. Receptor expression and responsiveness of human dendritic cells to a defined set of CC and CXC chemokines. J. Immunol. 159: 1993-2000.
43. Yang, D, Q.Chen, O. Chertov, and J. Oppenheim. 2000. Human neutrophil defensins selectively chemoattract naive T and immature dendritic cells. J. Leukoc. Biol. 68: 9-14.
44. Yang, D., O. Chertov, S. Bykovskaia, Q. Chen, M. Buffo, J. Shogan, M. Anderson, J. Schroder, J. Wang, O. Howard, and J. Oppenheim. 1999. Beta-defensins: linking innate and adaptive immunity through dendritic and T cell CCR6. Science 286: 525-528.

## Claims

1. A mucoadhesive pharmaceutical composition comprising an acrylic acid containing polymer and a chemoattractant wherein the pH of the composition is 6 or less.

2. A composition according to Claim 1 wherein the chemoattractant is granulocyte macrophage - colony stimulating factor (GM-CSF, molgramostim), MIP3α (macrophage inflamatory proteine 3α), HβD2 (human β defensin 2), HNP2 (human neutrophil defensin 2) and/or MCP-1 (monocyte chemotactic protein-1).

3. A composition according to Claim 1 or Claim 2 wherein the acrylic acid containing polymer is polycarbophil.

4. A composition according to any one of the preceding claims wherein the polymer is in the form of a hydrogel.

5. A composition according to any one of the preceding claims wherein the pH of the composition is preferably 5.5 or less.

6. A composition according to any one of the preceding claims wherein the pH of the composition is from 5 to 5.5.

7. A composition according to any one of the preceding claims for use in the treatment of a squamous mucosa, preferably in the treatment of an anogenital or oral disease, especially human papilloma virus.

8. Use of a composition according to any one of claims 1 to 6 in the manufacture of a medicament for use in the treatment of a squamous mucosa, preferably in the treatment of an anogenital or oral disease, especially human papilloma virus.

## Patentansprüche

1. Mukoadhäsive pharmazeutische Zusammensetzung, die ein acrylsäurehaltiges Polymer und ein Chemoattraktant umfaßt, wobei der pH-Wert der Zusammensetzung 6 oder weniger beträgt.

2. Zusammensetzung nach Anspruch 1, wobei es sich bei dem Chemoattraktant um den Granulocyten-Makrophagenkoloniestimulierenden Faktor (GM-CSF, Molgramostim), MIP3α (Makrophagen-Entzündungsprotein 3α), HβD2 (humanes β-Defensin 2), HNP2 (humanes neutrophiles Defensin 2) und/oder MCP-1 (Monocyten-chemotaktisches Protein-1) handelt.

3. Zusammensetzung nach Anspruch 1 oder Anspruch 2, wobei es sich bei dem acrylsäurehaltigen Polymer um Polycarbophil handelt.

4. Zusammensetzung nach einem der vorhergehenden Ansprüche, wobei das Polymer in Form eines Hydrogels vorliegt.

5. Zusammensetzung nach einem der vorhergehenden Ansprüche, wobei der pH-Wert der Zusammensetzung vorzugsweise 5,5 oder weniger beträgt.

6. Zusammensetzung nach einem der vorhergehenden Ansprüche, wobei der pH-Wert der Zusammensetzung von 5 bis 5,5 beträgt.

7. Zusammensetzung nach einem der vorhergehenden Ansprüche für die Verwendung bei der Behandlung einer squamösen Mukosa, vorzugsweise für die Behandlung einer Krankheit des Anogenitalbereichs oder einer Mundkrankheit, insbesondere des menschlichen Papilloma-Virus.

8. Verwendung einer Zusammensetzung nach einem der Ansprüche 1 bis 6 bei der Herstellung eines Arzneimittels für die Verwendung bei der Behandlung einer squamösen Mukosa, vorzugsweise für die Behandlung einer Krankheit des Anogenitalbereichs oder einer Mundkrankheit, insbesondere des menschlichen PapillomaVirus.

## Revendications

1. Composition pharmaceutique mucoadhésive comprenant un polymère contenant un acide acrylique et un chimioattracteur, le pH de cette composition étant de 6 ou moins.

2. Composition selon la revendication 1, le chimioattracteur étant le facteur stimulant de colonies de granulocytes-macrophages (GM-CSF, molgramostime), MIP3α, (protéine 3α inflammatoire du macrophage), HβD2 (défensine 2 β humaine), HNP2 (défensine neutrophile 2 humaine) et/ou MCP-1 (protéine chimiotactique de monocytes type 1).

3. Composition selon la revendication 1 ou la revendication 2, le polymére contenant de l'acide acrylique étant le polycarbophile.

4. Composition selon n'importe laquelle des revendications précédentes, le polymère étant sous la forme d'un hydrogel.

5. Composition selon n'importe laquelle des revendications précédentes, le pH de la composition étant de préférence de 5,5 ou moins.

6. Composition selon n'importe laquelle des revendications précédentes, le pH de la composition étant de 5 à 5,5.

7. Composition selon n'importe laquelle des revendications précédentes destinée au traitement d'une muqueuse squameuse, de préférence pour le traitement d'une maladie ano-génitale ou orale, en particulier due au papillomavirus humain

8. Utilisation d'une composition selon n'importe laquelle des revendications 1 à 6 dans la fabrication d'un médicament destiné au traitement d'une muqueuse squameuse, de préférence pour le traitement d'une maladie ano-génitale ou orale, en particulier due au papillomavirus humain.
